# EUROPEAN PATENT APPLICATION

(11) **EP 4 417 188 A1**
(43) Date of publication of application: **21.08.2024**
(21) Application number: 23157132.4
(22) Date of filing: 16.02.2023
(51) Int. Cl.: A61K 8/49, A61Q 1/02, C07D 311/94

(54) **GENIPIN DERIVATIVES**

(71) Applicant: inkbox ink Inc., Toronto, ON M5V 3G8 (CA)
(72) Inventor: CAPUTO, Christopher B., Toronto, ON, M5V 3G8 (CA); MANHAS, Sanjay, Toronto, ON, M5V 3G8 (CA); MALLOV, Ian, Toronto, ON, M5V 3G8 (CA); NLEND, Ingrid Um, Toronto, ON, M5V 3G8 (CA); JEEVA, Fiona, Toronto, ON, M5V 3G8 (CA); HANDLEY, Tyler J., Toronto, ON, M5V 3G8 (CA); GARRARD, Charley Nicole, Toronto, ON, M5V 3G8 (CA)
(74) Representative: Peterreins Schley

(57) **Abstract**

The present application relates to topical applications comprising novel genipin derivatives.

## Description

### TECHNICAL FIELD

The present disclosure relates to colored genipin derivatives which are attachable to the skin. The genipin derivatives are useful, for example, as colorants in semi-permanent tattoos.

### BACKGROUND

Temporary or semi-permanent tattoo or body inks have been used throughout human history to decorate the body. Generally, temporary or semi-permanent tattoos are transferred to the skin through the direct exposure of the skin to tattoo ink over a designated incubation period. The quality of the transferred image and its duration on the skin can depend on the ink distribution profile in the outer layer of the skin called the stratum corneum. Current manufacturing processes for prefabricated tattoo designs include the utilization of topical formulations in many forms, including flexographic or gravure printing inks, stencils, and inkjet printers.

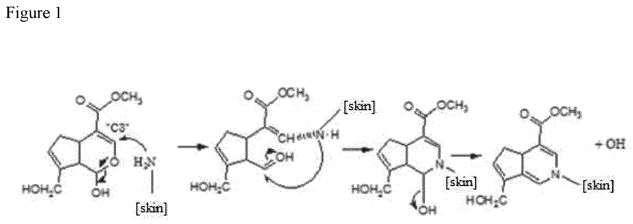

Genipin is particularly beneficial for use in such semi-permanent tattoos since it chemically binds to the skin (mammalian, in particular human skin) in a long-lasting fashion with an intense blue color. One model representing a potential mechanism of the chemical binding and color development on the skin is shown in Figure 1. As can be seen in Figure 1, genipin is believed to form a colored conjugated system when chemically binding to proteinaceous amino groups of the skin. The color of genipin - when bound to skin - is an intense dark blue.

However, it would be desirable to also provide semi-permanent tattoos in other but similarly intense colors and/or in other colors but of improved intensity. In practice, the design of a genipin derivative having a specific color can be challenging for a number of reasons: First, even despite the advances in calculating UV/vis-absorption spectra, it is very difficult to accurately predict the exact color impression and intensity for a given genipin derivative. Second, while the general principles of how conjugated systems and chemical moieties have an impact on UV/vis absorption are well-established, designing a genipin derivative having a desired color impression in a trial and error approach is time consuming and burdensome. The design of a specific genipin color is further burdened by the fact that the genipin core, when bound to skin, provides a first chromophore whereas other moieties on the genipin derivative provide further chromophoric systems. The resulting color impression is determined by the combined absorption of all chromophoric systems. Third, obtaining a desired color not only depends on providing absorption at a given wavelength but also depends on providing the appropriate amount of absorption at said wavelength. In practice, realizing these two goals (absorption at a given wavelength and intensity of absorption at said wavelength) with the same chemical chromophores can be challenging.

These challenges are further aggravated when desiring the provision of an intense yellow color: Depending on eye sensitivity, the human eye generally perceives pure yellow, i.e. without greenish or orange contributions, only in a very narrow wavelength range, namely from between about 566 to about 589 nm. Thus, absorption must be tailored around this wavelength region, more specifically in the vis-range above about 589 nm and in the vis-range below about 566 nm. At the same time, yellow is an important color since it is a primary color and useful in realizing many blended colors.

For these reasons, it would be desirable to have a genipin derivative which provides a suitable basis for conveniently fine-tuning the color impression and intensity, in particular for a yellow derivative.

### SUMMARY

In a first aspect, the present disclosure relates to a topical composition comprising a compound of formula (I), 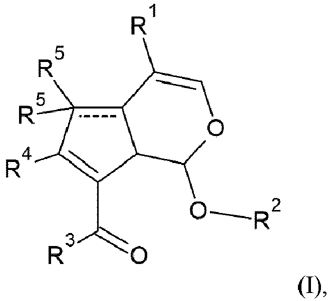 or a tautomer and/or a pharmaceutically acceptable salt thereof, wherein:
R¹ represents an optionally substituted C₁-C₃₀ moiety;
R² represents hydrogen or a protective group hydrolysable under physiological conditions after application of the topical composition onto skin;
R³ represents an optionally substituted C₁-C₃₀ moiety;
R⁴ represents hydrogen or an optionally substituted C₁-C₃₀ moiety;
wherein 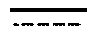 represents a single bond and both R⁵ independently from each other represent hydrogen or an optionally substituted C₁-C₃₀ moiety or wherein 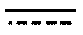 represents a double bond and one of R⁵ is absent and the other of R⁵ represents hydrogen or an optionally substituted C₁-C₃₀ moiety; and an excipient suitable for topical administration.

In some embodiments, the optionally substituted C₁-C₃₀ moiety represented by R¹ is a C₁-C₃₀ moiety comprising 1 to 30, more specifically 1 to 16, and in particular 1 to 12, carbon atoms; 0 to 12, more specifically 0 to 8, and in particular 0 to 6 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms.

In some embodiments, R¹ represents -COOH, R⁶ or -C(=O)-R⁶, wherein R⁶ represents optionally substituted C₁-C₁₂-alkyl, C₂-C₁₂-alkenyl, C₂-C₁₂-alkinyl, C₆-C₁₆-aryl or C₃-C₁₂-heteroaryl, O-C₁-C₁₂-alkyl, O-C₂-C₁₂-alkenyl, O-C₂-C₁₂-alkinyl, O-C₆-C₁₆-aryl or O-C₃-C₁₂-heteroaryl.

In some embodiments, R¹ represents COOH, C(=O)-O-C₁-C₁₂-alkyl or C(=O)-O-C₆-C₁₆-aryl, and in particular COOH or C(=O)-O-C₁-C₄-alkyl.

In some embodiments, the compound of formula (I) is a compound of formula (II), 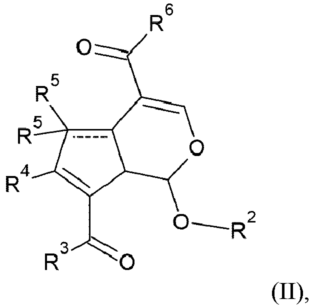 or a tautomer and/or a pharmaceutically acceptable salt thereof,
wherein R⁶ represents hydroxyl or an optionally substituted C₁-C₃₀ moiety; and wherein R², R³, R⁴, R⁵ and 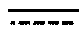 are defined as indicated above for formula (I).

In some embodiments, R² represents hydrogen or a C₁-C₆ acyl, and in particular hydrogen.

In some embodiments, R³ represents an optionally substituted C₁-C₁₂-alkyl, optionally substituted C₂-C₁₂-alkenyl, optionally substituted C₂-C₁₂-alkinyl, optionally substituted C₆-C₁₆-aryl or optionally substituted C₃-C₁₂-heteroaryl.

In some embodiments, R³ represents:
a) C₁-C₁₂-alkyl which is optionally substituted with the proviso that the optional substituents contain, in total, between 0 and 12, more specifically 0 to 8, and in particular 0 to 6, carbon atoms; between 0 to 8, more specifically 0 to 6, and in particular 0 to 4 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms;
b) C₁-C₁₂-alkenyl which is optionally substituted with the proviso that the optional substituents contain, in total, between 0 and 12, more specifically 0 to 8, and in particular 0 to 6, carbon atoms; between 0 to 8, more specifically 0 to 6, and in particular 0 to 4 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms;
c) C₁-C₁₂-alkinyl which is optionally substituted with the proviso that the optional substituents contain, in total, between 0 and 12, more specifically 0 to 8, and in particular 0 to 6, carbon atoms; between 0 to 8, more specifically 0 to 6, and in particular 0 to 4 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms;
d) C₆-C₁₆-aryl which is optionally substituted with the proviso that the optional substituents contain, in total, between 0 and 12, more specifically 0 to 8, and in particular 0 to 6, carbon atoms; between 0 to 8, more specifically 0 to 6, and in particular 0 to 4 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms; or
e) C₃-C₁₂-heteroaryl, comprising one, two, three or four heteroatoms selected from the group consisting of O, N and S, wherein the C₃-C₁₂-heteroaryl is optionally substituted with the proviso that the optional substituents contain, in total, between 0 and 12, more specifically 0 to 8, and in particular 0 to 6, carbon atoms; between 0 to 8, more specifically 0 to 6, and in particular 0 to 4 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms.

In some embodiments, R³ represents C₆-C₁₆-aryl which is optionally substituted by 1 to 5, more specifically 1, 2 or 3 substituent(s), and in particular 1 substituent, wherein each of said substituents is independently from each other selected from the group consisting of:
C₁-C₆-alkyl, more specifically C₁-C₄-alkyl, and in particular methyl, ethyl and tert-butyl;
C₁-C₄-haloalkyl, more specifically C₁-C₄-fluoroalkyl, and in particular trifluoromethyl;
O-C₁-C₆-alkyl, more specifically O-C₁-C₄-alkyl, and in particular methoxy or ethoxy;
O-C₂-C₁₂-alkenyl, more specifically O-C₂-C₇-alkenyl, and in particular O-C₂-C₅-alkenyl;
(C=O)-O-C₁-C₆-alkyl, more specifically (C=O)-O-C₁-C₄-alkyl;
O-(C=O)-C₁-C₆-alkyl, more specifically O-(C=O)-C₁-C₄-alkyl;
halogen, more specifically chloro or fluoro, and in particular fluoro; nitro;
NR⁶₂, wherein each of R⁶ independently from each other represents hydrogen, C₁-C₆ alkyl or C₆-C₁₂ aryl;
NH(CO)R⁶, wherein R⁶ represents C₁-C₆ alkyl or C₆-C₁₂ aryl;
(C=O)NR⁶₂, wherein each of R⁶ independently from each other represents hydrogen, C₁-C₆ alkyl or C₆-C₁₂ aryl; and
S-C₁-C₆-alkyl, more specifically S-C₁-C₄-alkyl, and in particular methylthio or ethylthio.

In some embodiments, R³ represents C₆-C₁₆-aryl which is optionally substituted by 1 to 5, more specifically 1, 2 or 3 substituent(s), and in particular 1 substituent, wherein each of said substituents is independently from each other selected from the group consisting of:
C₁-C₆-alkyl, more specifically C₁-C₄-alkyl, and in particular methyl, ethyl and tert-butyl;
C₁-C₄-haloalkyl, more specifically C₁-C₄-fluoroalkyl, and in particular trifluoromethyl;
O-C₁-C₆-alkyl, more specifically O-C₁-C₄-alkyl, and in particular methoxy or ethoxy;
halogen, more specifically chloro or fluoro, and in particular fluoro; and nitro.

In some embodiments, R³ represents phenyl which is substituted by 1, 2 or 3 substituent(s), wherein each of said substituents is independently from each other selected from the group consisting of:
C₁-C₆-alkyl, more specifically C₁-C₄-alkyl, and in particular methyl, ethyl and tert-butyl;
C₁-C₄-haloalkyl, more specifically C₁-C₄-fluoroalkyl, and in particular trifluoromethyl;
O-C₁-C₆-alkyl, more specifically O-C₁-C₄-alkyl, and in particular methoxy or ethoxy;
halogen, more specifically chloro or fluoro, and in particular fluoro; and nitro.

In some embodiments, R³ represents phenyl which is substituted by 1, 2 or 3 fluorine atoms and/or 1, 2 or 3 CF₃ groups, more specifically wherein R³ represents phenyl which is substituted by 1 fluorine atom or 1 CF₃ group, in particular by 1 fluorine atom, in the para-position, or by 2 fluorine atoms or 2 CF₃ groups, in particular by two fluorine atoms or by two CF₃ groups, in the meta-position.

In some embodiments, R³ represents phenyl which is monosubstituted by fluoro, a CF₃ group or O-C₁-C₄-alkyl in the ortho-, meta- and/or para-position, in particular monosubstituted by fluoro or CF₃ group, in particular fluoro, in the para-position.

In some embodiments, R³ represents one of the following moieties:

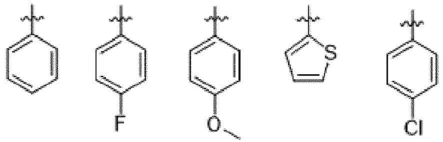

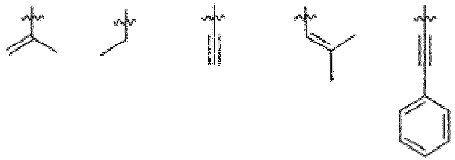

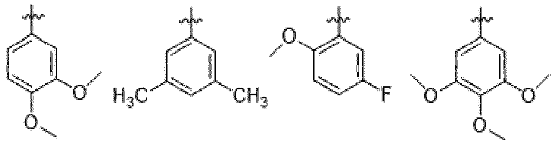

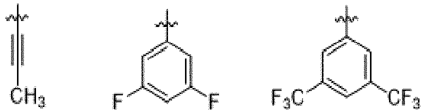

In some embodiments, the optionally substituted C₁-C₃₀ moiety represented by R⁴ is a C₁-C₃₀ moiety comprising 1 to 30, more specifically 1 to 16, and in particular 1 to 12, carbon atoms; 0 to 12, more specifically 0 to 8, and in particular 0 to 6 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms.

In some embodiments, R⁴ represents hydrogen, C₁-C₆ alkyl or C₆-C₁₆ aryl; more specifically hydrogen or C₁-C₄ alkyl; and in particular hydrogen.

In some embodiments, in formula (I) or (II), 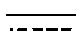 represents a single bond and both R⁵ independently from each other represent hydrogen, C₁-C₆ alkyl or C₆-C₁₆ aryl; more specifically hydrogen or C₁-C₄ alkyl; and in particular hydrogen.

In some embodiments, in formula (I) or (II), 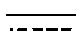 represents a double bond and one R⁵ is absent and the other R⁵ represents hydrogen, C₁-C₆ alkyl or C₆-C₁₆ aryl; more specifically hydrogen or C₁-C₄ alkyl; and in particular hydrogen.

In some embodiments, the compound of formula (I) is a compound of formula (III), 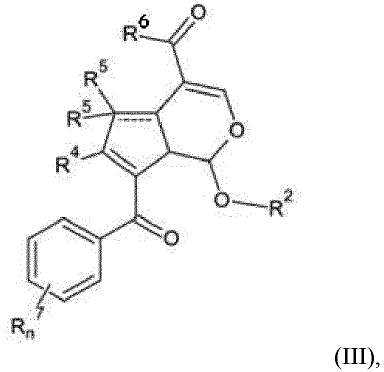
wherein R², R⁴, R⁵ and R⁶ are as defined for formula (I), wherein 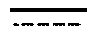 is as defined for formula (I), and wherein Rₙ⁷ represents n substituents R⁷,
wherein n is an integer between 0 and 5, more specifically between 0 and 3, and in particular 1, and wherein n being 0 means that R⁷ is absent,
wherein independently from each other R⁷ represents a moiety selected from the group consisting of:
   C₁-C₆-alkyl, more specifically C₁-C₄-alkyl, and in particular methyl, ethyl and tert-butyl;
   C₁-C₄-haloalkyl, more specifically C₁-C₄-fluoroalkyl, and in particular trifluoromethyl;
   O-C₁-C₆-alkyl, more specifically O-C₁-C₄-alkyl, and in particular methoxy or ethoxy;
   O-C₂-C₁₂-alkenyl, more specifically O-C₂-C₇-alkenyl, and in particular O-C₂-C₅-alkenyl; (C=O)-O-C₁-C₆-alkyl, more specifically (C=O)-O-C₁-C₄-alkyl;
   O-(C=O)-C₁-C₆-alkyl, more specifically O-(C=O)-C₁-C₄-alkyl;
   halogen, more specifically chloro or fluoro, and in particular fluoro; nitro;
   NR⁶₂, wherein each of R⁶ independently from each other represents hydrogen, C₁-C₆ alkyl or C₆-C₁₂ aryl;
   NH(CO)R⁶, wherein R⁶ represents C₁-C₆ alkyl or C₆-C₁₂ aryl;
   (C=O)NR⁶₂, wherein each of R⁶ independently from each other represents hydrogen, C₁-C₆ alkyl or C₆-C₁₂ aryl; and
   S-C₁-C₆-alkyl, more specifically S-C₁-C₄-alkyl, and in particular methylthio or ethylthio.

In some embodiments, in formula (III), n is 1, 2 or 3 and the R⁷ substituent(s) is/are in the ortho- and/or para-position(s). In some embodiments, n is 1 and R⁷ is in ortho- or para-position.

In some embodiments, in formula (III), each instance of R⁷ represents, independently from each other, a moiety selected from the group consisting of: O-C₁-C₆-alkyl, more specifically O-C₁-C₄-alkyl, and in particular methoxy or ethoxy; halogen, more specifically chloro or fluoro, and in particular fluoro; C₁-C₄-haloalkyl, more specifically C₁-C₄-fluoroalkyl, and in particular trifluoromethyl; and nitro.

In some embodiments, the compound of formula (I) is a compound of formula (IV), 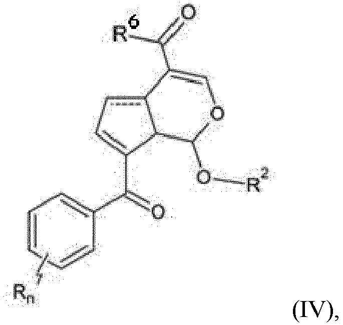 wherein R² and R⁶ are as defined for formula (I), wherein 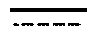 is as defined for formula (I), and wherein Rₙ⁷ is as defined above for formula (III).

In some embodiments, the compound of formula (I) is a compound of formula (V), 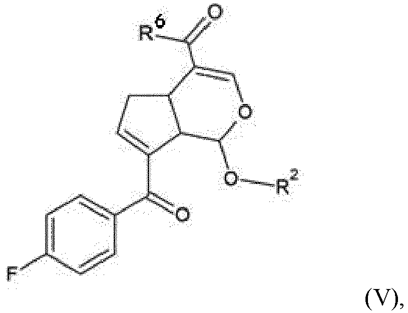 wherein R² and R⁶ are as defined for formula (I).

In some embodiments, the composition is in the form of a solid, a liquid, an ink, a gel, a patch for transferring the composition onto the skin, or a substrate carrier comprising the topical composition.

In some embodiments, the excipient suitable for topical administration comprises one or more excipients selected from water, ethanol, isopropanol, n-propanol, ethylene glycol, diethylene glycol, a propylene glycol, glycerol, and mixtures thereof.

In some embodiments, the composition comprises a thickening agent and/or a film-forming agent.

In some embodiments, the compound of formula (I), after conversion with lysine to a corresponding compound of formula (VI), 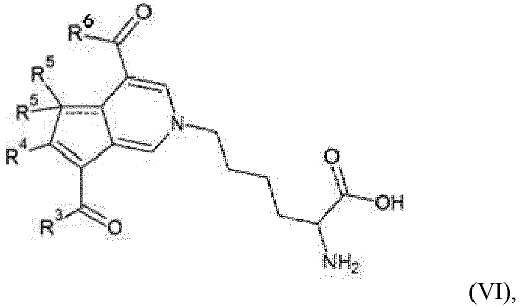
wherein R³, R⁴, R⁵ and R⁶ are as defined for formula (I), and wherein 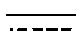 is as defined for formula (I),
absorbs light at a wavelength between about 380 nm and about 565 nm, more specifically between about 380 nm and about 568 nm, and in particular between about 380 nm and about 570 nm; wherein the corresponding compound of formula (VI) optionally also absorbs light at a wavelength between about 590 nm and about 750 nm, more specifically between about 587 nm and about 750 nm, and in particular between about 585 nm and about 750 nm; and
wherein the corresponding compound of formula (VI) optionally does not absorb light at a wavelength between about 566 nm and about 589 nm, more specifically between about 569 nm and about 586 nm, and in particular between about 571 nm and about 584 nm.

In some embodiments, the composition further comprises genipin or a genipin derivative, more specifically in relative molar amounts of 0.05 to 20 with respect to the amount of the compound of formula (I).

In some embodiments, the composition further comprises an antioxidant.

In a second aspect, the present disclosure relates to a storage unit comprising the topical composition according to any embodiment of the first aspect, wherein the storage unit is protected against ingress of oxygen or contains an oxygen scavenger.

In a third aspect, the present disclosure relates to a process of preparing the topical composition of any of the embodiments of the first aspect, the process comprising mixing a compound of formula (I), as defined above, with an excipient suitable for topical administration.

In a fourth aspect, the present disclosure relates to process of mixing a compound of formula (I), as defined above, with genipin or a genipin derivative.

### DESCRIPTION OF THE FIGURE

Figure 1 shows one potential binding mechanism of genipin to skin, in this case to an amino group of a skin macromolecule such as collagen or keratine.

### DETAILED DESCRIPTION

Provided herein are topical compositions comprising semi-permanent colorants and/or semi-permanent colorant precursors that include derivatives of genipin. As used herein, a "topical composition" means a composition that is suitable (i.e. cosmetically suitable) to be applied on skin, more specifically on mammalian skin, in particular human skin. As used herein, a "semi-permanent colorant" refers to a colorant that penetrates one or more layers of the skin and is unable to be removed from the skin without physical disruption or natural desquamation of the skin. In some embodiments, a semi-permanent colorant can include a colorant precursor of a genipin derivative that expresses color upon reaction with one or more other molecules on e.g. the skin. In some embodiments, a semi-permanent colorant may penetrate the stratum corneum and react with other molecules present in the stratum corneum, such that the semi-permanent colorant is immobilized within the stratum corneum. For example, the semi-permanent colorant may react with e.g. collagen or keratin found in the stratum corneum. The semi-permanent colorant may penetrate the stratum corneum and the colorant residence time may be determined by the natural skin desquamation process. In some embodiments, the semi-permanent colorant cannot be washed off, for example, by water, soap, and/or isopropanol.

In a first aspect, the present disclosure relates to a topical composition comprising a compound of formula (I), 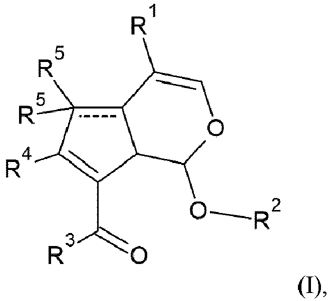 or a tautomer and/or a pharmaceutically acceptable salt thereof, wherein:
R¹ represents an optionally substituted C₁-C₃₀ moiety;
R² represents hydrogen or a protective group hydrolysable under physiological conditions after application of the topical composition onto skin;
R³ represents an optionally substituted C₁-C₃₀ moiety;
R⁴ represents hydrogen or an optionally substituted C₁-C₃₀ moiety;
wherein 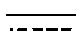 represents a single bond and both R⁵ independently from each other represent hydrogen or an optionally substituted C₁-C₃₀ moiety or wherein 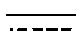 represents a double bond and one of R⁵ is absent and the other of R⁵ represents hydrogen or an optionally substituted C₁-C₃₀ moiety; and an excipient suitable for topical administration.

The present inventors have surprisingly found that providing a carbonyl-based substituent to the conjugated core system of a genipin derivative (when bound to amino groups as found on the skin) provides a vibrant and intense color impression to the chromophoric system of the genipin derivative. As will be described in the experimental section, the color impression is substantially more vibrant than a genipin derivative carrying an aldehyde-based substituent in place of the carbonyl-based substituent (intense vibrant yellow in comparison to a pale yellow). Moreover, while both aldehyde- and carbonyl-substituents may provide similar color impressions, it is believed that the carbonyl-based genipin derivatives will be more stable and possibly longer lasting (when applied onto the skin) than the corresponding aldehyde analogues.

When referring to R¹ being an optionally substituted C₁-C₃₀ moiety, it should be understood that said moiety may optionally comprise other atoms but that said optional substituents may not result in the total carbon number of the moiety exceeding 30. In some embodiments, the optionally substituted C₁-C₃₀ moiety may be an (optionally substituted) C₁-C₁₈ moiety or an (optionally substituted) C₁-C₁₂ moiety.

When referring in context of R² to protective groups hydrolysable under physiological conditions after application of the topical composition onto the skin: Such groups are readily known to the skilled person and include, in particular acyl moieties such as optionally substituted acyl moieties comprising 1 to 30, more specifically 1 to 16, and in particular 1 to 12, carbon atoms; 0 to 12, more specifically 0 to 8, and in particular 0 to 6 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms. Specific examples include C₁-C₁₈ acyl moieties, more specifically C₁-C₁₂ acyl moieties and in particular C₁-C₄ acyl moieties. When referring in this context to "physiological conditions", it should be understood that this in particular refers to the pH conditions encountered at the locus where the compound of formula (I) is supposed to bind to proteins in e.g. the stratum corneum and/or the epidermis of the (mammalian, in particular human) subject to be treated. Additionally or alternatively, the term "protective groups hydrolysable under physiological conditions after application of the topical composition" refers to a group which is hydrolysed when placing 0.1 mol/l of the compound of formula (I) in aqueous solution having a pH of about 5 and further containing 0.1 mol/l lysine at 37°C for 2 hours, wherein the group in question qualifies as "protective groups hydrolysable under physiological conditions after application of the topical composition" if a notable amount of a 1,4-dihydropyridine derivative (e.g. conversion yield greater than 10 mol%) is formed. Additionally or alternatively, a compound of formula (I) contains a "protective groups hydrolysable under physiological conditions after application of the topical composition" if the compound (or a topical composition containing the compound) cannot be comprehensively washed off from (explanted) porcine skin by water, soap, and/or isopropanol after incubating the compound (or the topical composition comprising the compound) on the porcine skin at e.g. 37°C for e.g. 2 hours.

In some embodiments, the optionally substituted C₁-C₃₀ moiety represented by R¹ is a moiety comprising 1 to 30, more specifically 1 to 16, and in particular 1 to 12, carbon atoms; 0 to 12, more specifically 0 to 8, and in particular 0 to 6 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms. The aforementioned numbers refer to the total number of the carbon, oxygen, nitrogen sulfur and halogen, respectively. It should be understood that said optionally substituted C₁-C₃₀ moiety may optionally comprise other atoms and optionally further substituents, but that the aforementioned numbers of carbon, oxygen, nitrogen sulfur and halogen may not be exceeded even in the presence of such optional further substituents. It should also be understood that halogen refers to, in particular, fluoro, chloro and bromo.

In some embodiments, R¹ represents -COOH, R⁶ or -C(=O)-R⁶, wherein R⁶ represents optionally substituted C₁-C₁₂-alkyl, C₂-C₁₂-alkenyl, C₂-C₁₂-alkinyl, C₆-C₁₆-aryl or C₃-C₁₂-heteroaryl, O-C₁-C₁₂-alkyl, O-C₂-C₁₂-alkenyl, O-C₂-C₁₂-alkinyl, O-C₆-C₁₆-aryl or O-C₃-C₁₂-heteroaryl.

In some embodiments, R¹ represents COOH, C(=O)-O-C₁-C₁₂-alkyl or C(=O)-O-C₆-C₁₆-aryl, and in particular COOH or C(=O)-O-C₁-C₄-alkyl.

In some embodiments, the compound of formula (I) is a compound of formula (II), 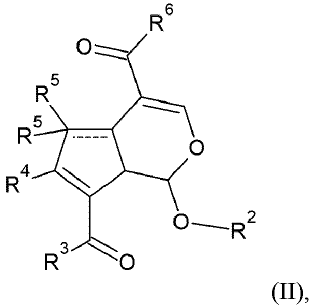 or a tautomer and/or a pharmaceutically acceptable salt thereof,
wherein R⁶ represents hydroxyl or an optionally substituted C₁-C₃₀ moiety; and wherein R², R³, R⁴, R⁵ and 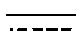 are defined as indicated above for formula (I).

In some embodiments, R² represents hydrogen or a C₁-C₆ acyl, and in particular hydrogen.

In some embodiments, R³ represents an optionally substituted C₁-C₁₂-alkyl, optionally substituted C₂-C₁₂-alkenyl, optionally substituted C₂-C₁₂-alkinyl, optionally substituted C₆-C₁₆-aryl or optionally substituted C₃-C₁₂-heteroaryl.

In some embodiments, the R³ represents:
a) C₁-C₁₂-alkyl which is optionally substituted with the proviso that the optional substituents contain, in total, between 0 and 12, more specifically 0 to 8, and in particular 0 to 6, carbon atoms; between 0 to 8, more specifically 0 to 6, and in particular 0 to 4 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms, more specifically fluoro, chloro or bromo and in particular fluoro;
b) C₁-C₁₂-alkenyl which is optionally substituted with the proviso that the optional substituents contain, in total, between 0 and 12, more specifically 0 to 8, and in particular 0 to 6, carbon atoms; between 0 to 8, more specifically 0 to 6, and in particular 0 to 4 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms, more specifically fluoro, chloro or bromo and in particular fluoro;
c) C₁-C₁₂-alkinyl which is optionally substituted with the proviso that the optional substituents contain, in total, between 0 and 12, more specifically 0 to 8, and in particular 0 to 6, carbon atoms; between 0 to 8, more specifically 0 to 6, and in particular 0 to 4 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms, more specifically fluoro, chloro or bromo and in particular fluoro;
d) C₆-C₁₆-aryl which is optionally substituted with the proviso that the optional substituents contain, in total, between 0 and 12, more specifically 0 to 8, and in particular 0 to 6, carbon atoms; between 0 to 8, more specifically 0 to 6, and in particular 0 to 4 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms, more specifically fluoro, chloro or bromo and in particular fluoro; or
e) C₃-C₁₂-heteroaryl, comprising one, two, three or four heteroatoms selected from the group consisting of O, N and S, wherein the C₃-C₁₂-heteroaryl is optionally substituted with the proviso that the optional substituents contain, in total, between 0 and 12, more specifically 0 to 8, and in particular 0 to 6, carbon atoms; between 0 to 8, more specifically 0 to 6, and in particular 0 to 4 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms, more specifically fluoro, chloro or bromo and in particular fluoro.

In some embodiments, it may be particularly advantageous that R³ represents C₆-C₁₆-aryl which is optionally further substituted with the proviso that the optional substituents contain, in total, between 0 and 12, more specifically 0 to 8, and in particular 0 to 6, carbon atoms; between 0 to 8, more specifically 0 to 6, and in particular 0 to 4 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms, wherein the halogen atoms, more specifically fluoro, chloro or bromo and in particular fluoro.

In some embodiments, it may be particularly advantageous that R³ represents C₃-C₁₂-heteroaryl comprising one, two, three or four heteroatoms selected from the group consisting of O, N and S, wherein the C₃-C₁₂-heteroaryl is optionally further substituted with the proviso that the optional substituents contain, in total, between 0 and 12, more specifically 0 to 8, and in particular 0 to 6, carbon atoms; between 0 to 8, more specifically 0 to 6, and in particular 0 to 4 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms, more specifically fluoro, chloro or bromo and in particular fluoro. The term "heteroaryl" as used herein refers to stable heterocyclic or polyheterocyclic aromatic moieties having 3 to 12 carbon ring atoms. As indicated above, the heteroaryl groups may be substituted or unsubstituted within the aforementioned limits. The heteroaryl may comprise one or more rings. Examples of typical heteroaryl rings include 5-membered monocyclic ring groups such as thienyl, pyrrolyl, imidazolyl, pyrazolyl, furyl, isothiazolyl, furazanyl, isoxazolyl, thiazolyl and the like; 6-membered monocyclic groups such as pyridyl, pyrazinyl, pyrimidinyl, pyridazinyl, triazinyl and the like; and polycyclic heterocyclic ring groups such as benzo[b]thienyl, naphtho[2,3-b]thienyl, thianthrenyl, isobenzofuranyl, chromenyl, xanthenyl, phenoxathienyl, indolizinyl, isoindolyl, indolyl, indazolyl, purinyl, isoquinolyl, quinolyl, phthalazinyl, naphthyridinyl, quinoxalinyl, quinazolinyl, benzothiazole, benzimidazole, tetrahydroquinoline cinnolinyl, pteridinyl, carbazolyl, beta-carbolinyl, phenanthridinyl, acridinyl, perimidinyl, phenanthrolinyl, phenazinyl, isothiazolyl, phenothiazinyl, phenoxazinyl, and the like (see e.g. Katritzky, Handbook of Heterocyclic Chemistry, 3^{rd} ed. 2010). Further specific examples of heteroaryl rings include 2-furanyl, 3-furanyl, N-imidazolyl, 2-imidazolyl, 4-imidazolyl, 5-imidazolyl, 3-isoxazolyl, 4-isoxazolyl, 5-isoxazolyl, 2-oxadiazolyl, 5-oxadiazolyl, 2-oxazolyl, 4-oxazolyl, 5-oxazolyl, 1-pyrrolyl, 2-pyrrolyl, 3-pyrrolyl, 2-pyridyl, 3-pyridyl, 4-pyridyl, 2-pyrimidyl, 4-pyrimidyl, 5-pyrimidyl, 3-pyridazinyl, 2-thiazolyl, 4- thiazolyl, 5-thiazolyl, 5-tetrazolyl, 2-triazolyl, 5-triazolyl, 2-thienyl, 3-thienyl, carbazolyl, benzimidazolyl, benzothienyl, benzofuranyl, indolyl, quinoiinyl, benzotriazolyl, benzothiazolyl, benzooxazolyl, benzimidazolyl, isoquinolinyl, indolyl, isoindolyl, acridinyl, or benzoisoxazolyl. Heteroaryl groups further include a group in which a heteroaromatic ring is fused to one or more aromatic or nonaromatic rings where the radical or point of attachment is on the heteroaromatic ring. Examples include tetrahydroquinoline, tetrahydroisoquinoline, and pyrido[3,4-d]pyrimidinyl, imidazo[1,2-a]pyrimidyl, imidazo[1,2-a]pyrazinyl, imidazo[1,2-a]pyiridinyl, imidazo[1,2-c]pyrimidyl, pyrazolo[1,5-a][1,3,5]triazinyl, pyrazolo[1,5-c]pyrimidyl, imidazo[1,2-b]pyridazinyl, imidazo[1,5-a]pyrimidyl, pyrazolo[1,5-b][1,2,4]triazine, quinolyl, isoquinolyl, quinoxalyl, imidazotriazinyl, pyrrolo[2,3-d]pyrimidyl, triazolopyrimidyl, pyridopyrazinyl.

In some embodiments, it may be particularly advantageous that R³ represents C₆-C₁₆-aryl which is optionally substituted by 1 to 5, more specifically 1, 2 or 3 substituent(s), and in particular 1 substituent, wherein each of said substituents is independently from each other selected from the group consisting of:
C₁-C₆-alkyl, more specifically C₁-C₄-alkyl, and in particular methyl, ethyl and tert-butyl;
C₁-C₄-haloalkyl, more specifically C₁-C₄-fluoroalkyl, and in particular trifluoromethyl;
O-C₁-C₆-alkyl, more specifically O-C₁-C₄-alkyl, and in particular methoxy or ethoxy;
O-C₂-C₁₂-alkenyl, more specifically O-C₂-C₇-alkenyl, and in particular O-C₂-C₅-alkenyl;
(C=O)-O-C₁-C₆-alkyl, more specifically (C=O)-O-C₁-C₄-alkyl;
O-(C=O)-C₁-C₆-alkyl, more specifically O-(C=O)-C₁-C₄-alkyl;
halogen, more specifically chloro or fluoro, and in particular fluoro; nitro;
NR⁶₂, wherein each of R⁶ independently from each other represents hydrogen, C₁-C₆ alkyl or C₆-C₁₂ aryl;
NH(CO)R⁶, wherein R⁶ represents C₁-C₆ alkyl or C₆-C₁₂ aryl;
(C=O)NR⁶₂, wherein each of R⁶ independently from each other represents hydrogen, C₁-C₆ alkyl or C₆-C₁₂ aryl; and
S-C₁-C₆-alkyl, more specifically S-C₁-C₄-alkyl, and in particular methylthio or ethylthio.

In some embodiments, it may be particularly advantageous that R³ represents C₆-C₁₂-aryl which is optionally substituted by 1 to 5, more specifically 1, 2 or 3 substituent(s), and in particular 1 substituent, wherein each of said substituents is independently from each other selected from the group consisting of:
C₁-C₆-alkyl, more specifically C₁-C₄-alkyl, and in particular methyl, ethyl and tert-butyl;
C₁-C₄-haloalkyl, more specifically C₁-C₄-fluoroalkyl, and in particular trifluoromethyl;
O-C₁-C₆-alkyl, more specifically O-C₁-C₄-alkyl, and in particular methoxy or ethoxy;
halogen, more specifically chloro or fluoro, and in particular fluoro; and nitro.

In some embodiments, it may be particularly advantageous that R³ represents phenyl which is substituted by 1, 2 or 3 substituent(s), wherein each of said substituents is independently from each other selected from the group consisting of:
C₁-C₆-alkyl, more specifically C₁-C₄-alkyl, and in particular methyl, ethyl and tert-butyl;
C₁-C₄-haloalkyl, more specifically C₁-C₄-fluoroalkyl, and in particular trifluoromethyl;
O-C₁-C₆-alkyl, more specifically O-C₁-C₄-alkyl, and in particular methoxy or ethoxy;
halogen, more specifically chloro or fluoro, and in particular fluoro; and nitro.

In some embodiments, R³ is substituted by 1, 2, 3, 4 or 5 fluorine atoms and/or fluorine-containing groups, in particular CF₃.

In some embodiments, R³ represents phenyl, naphthenyl, anthracenyl, pyrenyl, indenyl, thiophenyl, furanyl, benzoxazolyl or indolyl which is substituted by 1, 2, 3, 4 or 5 fluorine atoms and/or fluorine-containing groups, in particular CF₃.

In some embodiments, R³ represents phenyl which is substituted by 1, 2 or 3 fluorine atoms and/or 1, 2 or 3 CF₃ groups, more specifically wherein R³ represents phenyl which is substituted by 1 fluorine atom or 1 CF₃ group, in particular by 1 fluorine atom in the para-position, or by 2 fluorine atoms or 2 CF₃ groups, in particular by two fluorine atoms or by two CF₃ groups in the meta-position.

In some embodiments, R³ represents phenyl which is monosubstituted by fluoro or CF₃ group or O-C₁-C₄-alkyl in the ortho-, meta- and/or para-position, in particular monosubstituted by fluoro or CF₃ group, in particular fluoro, in the para-position.

In some embodiments, R³ represents one of the following moieties:

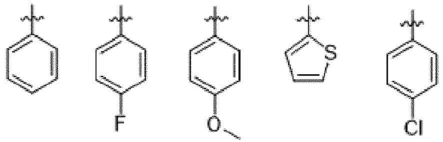

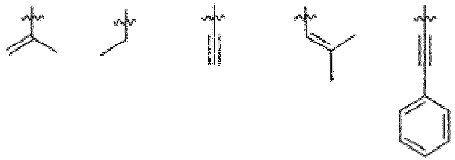

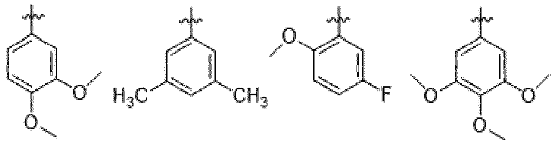

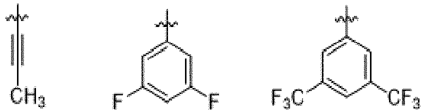

In some embodiments, R³ represents one of the following moieties:

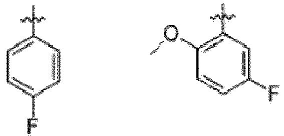

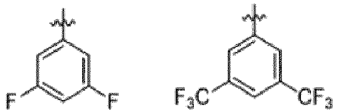

The fluoro-based substituent in the aforementioned embodiments may provide a hyperchromic effect (i.e. an increase in absorption) without causing a pronounced bathochromic or hypsochromic shift. For these reasons, genipin derivatives of the present disclosure carrying such substituents are of particular interest in providing genipin derivatives having intense colors and in facilitating the design of genipin derivatives having a desired color since the absorbance of the R³ moiety can be, to a degree, decoupled from the absorption wavelength.

In some embodiments, the optionally substituted C₁-C₃₀ moiety represented by R⁴ is a C₁-C₃₀ moiety comprising 1 to 30, more specifically 1 to 16, and in particular 1 to 12, carbon atoms; 0 to 12, more specifically 0 to 8, and in particular 0 to 6 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms. In some embodiments, the optionally substituted C₁-C₃₀ moiety may be an (optionally substituted) C₁-C₁₈ moiety or an (optionally substituted) C₁-C₁₂ moiety.

In some embodiments, the optionally substituted C₁-C₃₀ moiety represented by R⁴ is a moiety comprising 1 to 30, more specifically 1 to 16, and in particular 1 to 12, carbon atoms; 0 to 12, more specifically 0 to 8, and in particular 0 to 6 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms. The aforementioned numbers refer to the total number of the carbon, oxygen, nitrogen sulfur and halogen, respectively. It should be understood that said optionally substituted C₁-C₃₀ moiety may optionally comprise other atoms and optionally further substituents, but that the aforementioned numbers of carbon, oxygen, nitrogen sulfur and halogen may not be exceeded even in the presence of such optional further substituents. It should also be understood that halogen refers to, in particular, fluoro, chloro and bromo.

In some embodiments, R⁴ represents hydrogen, C₁-C₆ alkyl or C₆-C₁₆ aryl; more specifically hydrogen or C₁-C₄ alkyl; and in particular hydrogen.

In some embodiments, the optionally substituted C₁-C₃₀ moiety represented by each instance of R⁵ is (independently from each other) a moiety comprising 1 to 30, more specifically 1 to 16, and in particular 1 to 12, carbon atoms; 0 to 12, more specifically 0 to 8, and in particular 0 to 6 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms. The aforementioned numbers refer to the total number of the carbon, oxygen, nitrogen sulfur and halogen, respectively. It should be understood that said optionally substituted C₁-C₃₀ moiety may optionally comprise other atoms and optionally further substituents, but that the aforementioned numbers of carbon, oxygen, nitrogen sulfur and halogen may not be exceeded even in the presence of such optional further substituents. It should also be understood that halogen refers to, in particular, fluoro, chloro and bromo.

In some embodiments, in formula (I), 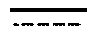 represents a single bond and both R⁵ independently from each other represent hydrogen, C₁-C₆ alkyl or C₆-C₁₆ aryl; more specifically hydrogen or C₁-C₄ alkyl; and in particular hydrogen.

In some embodiments, in formula (I), 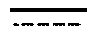 represents a double bond and one R⁵ is absent and the other R⁵ represents hydrogen, C₁-C₆ alkyl or C₆-C₁₆ aryl; more specifically hydrogen or C₁-C₄ alkyl; and in particular hydrogen.

In some embodiments, the compound of formula (I) is a compound of formula (III), 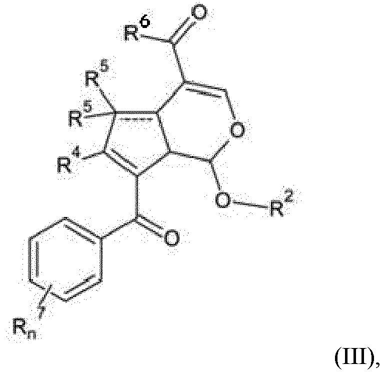 wherein R², R⁴, R⁵ and R⁶ are as defined for formula (I), wherein 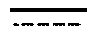 is as defined for formula (I), and wherein Rₙ⁷ represents n substituents R⁷, wherein n is an integer between 0 and 5, more specifically between 0 and 3, and in particular 1, and wherein n being 0 means that R⁷ is absent, wherein independently from each other R⁷ represents a moiety selected from the group consisting of:
C₁-C₆-alkyl, more specifically C₁-C₄-alkyl, and in particular methyl, ethyl and tert-butyl;
C₁-C₄-haloalkyl, more specifically C₁-C₄-fluoroalkyl, and in particular trifluoromethyl;
O-C₁-C₆-alkyl, more specifically O-C₁-C₄-alkyl, and in particular methoxy or ethoxy;
O-C₂-C₁₂-alkenyl, more specifically O-C₂-C₇-alkenyl, and in particular O-C₂-C₅-alkenyl;
(C=O)-O-C₁-C₆-alkyl, more specifically (C=O)-O-C₁-C₄-alkyl;
O-(C=O)-C₁-C₆-alkyl, more specifically O-(C=O)-C₁-C₄-alkyl;
halogen, more specifically chloro or fluoro, and in particular fluoro; nitro;
NR⁶₂, wherein each of R⁶ independently from each other represents hydrogen, C₁-C₆ alkyl or C₆-C₁₂ aryl;
NH(CO)R⁶, wherein R⁶ represents C₁-C₆ alkyl or C₆-C₁₂ aryl;
(C=O)NR⁶₂, wherein each of R⁶ independently from each other represents hydrogen, C₁-C₆ alkyl or C₆-C₁₂ aryl; and
S-C₁-C₆-alkyl, more specifically S-C₁-C₄-alkyl, and in particular methylthio or ethylthio.

In some embodiments, in formula (III), n is 1, 2 or 3 and the R⁷ substituent(s) is/are in the ortho- and/or para-position(s). In some embodiments, n is 1 and R⁷ is in ortho- or para-position.

In some embodiments, in formula (III), each instance of R⁷ represents, independently from each other, a moiety selected from the group consisting of: O-C₁-C₆-alkyl, more specifically O-C₁-C₄-alkyl, and in particular methoxy or ethoxy; halogen, more specifically chloro or fluoro, and in particular fluoro; C₁-C₄-haloalkyl, more specifically C₁-C₄-fluoroalkyl, and in particular trifluoromethyl; and nitro.

In some embodiments, the compound of formula (I) is a compound of formula (IV), 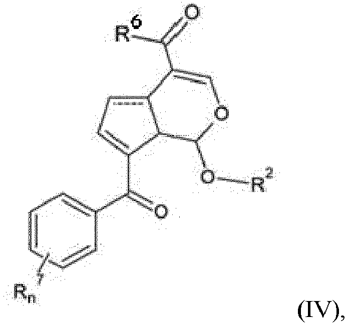 wherein R² and R⁶ are as defined for formula (I), wherein 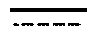 is as defined for formula (I), and wherein Rₙ⁷ is as defined above for formula (III).

In some embodiments, the compound of formula (I) is a compound of formula (V), 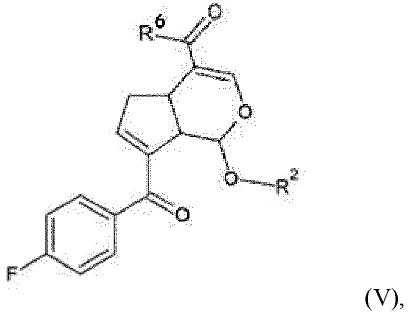 wherein R² and R⁶ are as defined for formula (I).

Generally, the aforementioned compositions can be formulated in any form known in the art for topical or cosmetic administration. Hence, the composition can be applied in any topical form, such as in the form of aerosol spray, cream, emulsion, solid, liquid, dispersion, foam, oil, gel, hydrogel, lotion, mousse, ointment, powder, patch, pomade, solution, pump spray, stick, towelette, soap, or other forms commonly employed in the art of topical administration and/or cosmetic/sunscreen and skin care formulation. The composition can also be water-resistant (e.g., waterproof).

In some embodiments, the composition is in the form of a solid, a liquid, an ink, a gel, a patch for transferring the composition onto skin, or a substrate carrier comprising the topical composition.

The compositions of this disclosure may contain any one of the compounds described herein in the range of 0.005wt.-% to 99wt.-% with the balance made up from the suitable excipients. The contemplated compositions may contain 0.001 wt.-%-99 wt.-% of any one of the compounds provided herein, in one embodiment 0.1-95 wt.-%, in another embodiment 75-85 wt.-%, in a further embodiment 20-80 wt.-%, wherein the balance may be made up of any excipient described herein, or any combination of these excipients.

In some embodiments, the topical composition comprises one or more additional ingredients, carriers or diluents including, but not limited to, absorbents, anti-irritants, anti-acne agents, preservatives, antioxidants, coloring agents/pigments, emollients (moisturizers), emulsifiers, film-forming/holding agents, fragrances, leave-on exfoliants, prescription drugs, preservatives, scrub agents, silicones, skin-identical/repairing agents, slip agents, additional conventional sunscreen actives (non-limiting examples of such sunscreens include titanium oxide and zinc oxide), surfactants/detergent cleansing agents, penetration enhancers, and thickeners. In particular, the composition may contain any acceptable excipient that is approved by FDA or EMA for such use. Examples of acceptable inactive excipients include retinyl palmitate, parabens, PEGs, fragrance, BHT, phthalates, phenoxyethanol, coconut oil, aloe vera, sunflower seed oil, calendula, almond oil, starch, gelatin, hemicellulose, arabinogalactan, agar, glycerol, lactic acid, propylene glycol, polyethylene glycol, polyvinylpyrrolidone, acrylates, acrylamides, copolymers of the foregoing, an amino acid such as arginine, alanine, or asparagine, ascorbic acid, citric acid, and mixtures thereof.

In some embodiments, the excipient suitable for topical administration comprises one or more excipients selected from water, ethanol, isopropanol, n-propanol, ethylene glycol, diethylene glycol, a propylene glycol, glycerol, and mixtures thereof.

In some embodiments, the composition comprises a thickening agent and/or a film-forming agent.

In some embodiments, the composition further comprises an antioxidant.

In some embodiments, the compound of formula (I), after conversion with lysine to a corresponding compound of formula (VI), 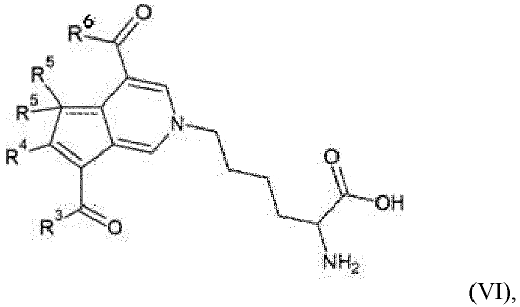
wherein R³, R⁴, R⁵ and R⁶ are as defined for formula (I), and wherein 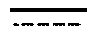 is as defined for formula (I),
absorbs light at a wavelength between about 380 nm and about 565 nm, more specifically between about 380 nm and about 568 nm, and in particular between about 380 nm and about 570 nm; wherein the corresponding compound of formula (VI) optionally also absorbs light at a wavelength between about 590 nm and about 750 nm, more specifically between about 587 nm and about 750 nm, and in particular between about 585 nm and about 750 nm; and
wherein the corresponding compound of formula (VI) optionally does not absorb light at a wavelength between about 566 nm and about 589 nm, more specifically between about 569 nm and about 586 nm, and in particular between about 571 nm and about 584 nm.

Regarding to the aforementioned absorption characteristics, it should be noted the human eye is most sensitive towards green light and, thus, absorption of light at a wavelength between about 380 nm and about 565 nm, more specifically between about 380 nm and about 568 nm, and in particular between about 380 nm and about 570 nm, removes, in particular, those parts from the vis-spectrum which would provide a green hue to the color and which to which the human eye is most receptive.

Optionally the aforementioned corresponding compound of formula (VI) does not absorb light at a wavelength between about 566 nm and about 589 nm, more specifically between about 569 nm and about 586 nm, and in particular between about 571 nm and about 584 nm, which corresponds to wavelengths which the human eye perceives as red or reddish. Since the human eye is substantially less sensitive to these wavelengths than to "green" wavelengths, a lower (relative) absorption is generally better tolerated in this wavelength region than in the "green" wavelength region.

Furthermore, the aforementioned corresponding compound of formula (VI) optionally absorbs light at a wavelength between about 590 nm and about 750 nm, more specifically between about 587 nm and about 750 nm, and in particular between about 585 nm and about 750 nm. This wavelength region is generally perceived by the human eye as yellow and the narrow bandwidth of wavelength reflected by this feature is a measure of the brilliance of the color (more narrow wavelength distributions are perceived as more brilliant by the human eye).

The measuring conditions for determining the absorption (or lack thereof) of the aforementioned corresponding compound of formula (VI) are not particularly limited. Exemplary conditions are typical conditions for UV/vis-spectrometry, e.g. between about 1-5 × 10⁻⁵ M solutions at about 20°C. The person of skill in the art is generally capable of interpreting a UV/vis-spectrum and determining whether absorption is present or not at a certain wavelength region. However, in case of need for a quantitative measure, the corresponding compound of formula (VI) may be considered to not if, in the wavelength region in question,a (normalized) coefficient of absorption is not higher than 0.4, specifically higher than 0.2 and in particular higher than 0.1).

In some embodiments, the composition further comprises an antioxidant.

In a second aspect, the present disclosure relates to a storage unit comprising the topical composition according to any embodiment of the first aspect, wherein the storage unit is protected against ingress of oxygen or contains an oxygen scavenger.

In a third aspect, the present disclosure relates to a process of preparing the topical composition of any of the embodiments of the first aspect, the process comprising mixing a compound of formula (I), as defined above, with an excipient suitable for topical administration.

In a fourth aspect, the present disclosure relates to process of mixing a compound of formula (I), as defined above, with genipin or a genipin derivative.

The term "genipin derivative" comprises any derivative of genipin and, in particular, any compound which comprises the following substituted chemical moiety of formula (VII), 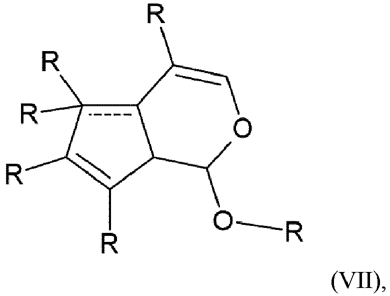 wherein R independently from each other represents H or a substituent and wherein 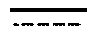 represents a single bond and both R attached to 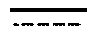 independently from each other represent hydrogen or a substituent or wherein 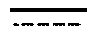 represents a double bond and one of R attached to 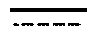 is absent and the other of R attached to 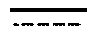 represents hydrogen or a substituent. In some embodiments, the genipin derivative is a compound of formula (I).

Compounds provided herein also include tautomeric forms. Tautomeric forms result from the swapping of a single bond with an adjacent double bond together with the concomitant migration of a proton. Tautomeric forms include prototropic tautomers which are isomeric protonation states having the same empirical formula and total charge. Example prototropic tautomers include ketone - enol pairs, amide - imidic acid pairs, lactam - lactim pairs, enamine - imine pairs, and annular forms where a proton can occupy two or more positions of a heterocyclic system, for example, 1Hand 3H-imidazole, 1H-, 2H- and 4H-1,2,4-triazole, 1H- and 2H-isoindole, and 1H- and 2H-pyrazole. Tautomeric forms can be in equilibrium or sterically locked into one form by appropriate substitution.

As used herein, a "salt" or "pharmaceutically acceptable salt" of a compound of any one of the formulae disclosed herein is formed between an acid and a basic group of the compound, such as an amino functional group, or a base and an acidic group of the compound, such as a carboxyl functional group. According to another embodiment, the compound is a pharmaceutically acceptable acid addition salt. In some embodiments, acids commonly employed to form pharmaceutically acceptable salts of the compounds of any one of the formulae include inorganic acids such as hydrogen bisulfide, hydrochloric acid, hydrobromic acid, hydroiodic acid, sulfuric acid and phosphoric acid, as well as organic acids such as para-toluenesulfonic acid, salicylic acid, tartaric acid, bitartaric acid, ascorbic acid, maleic acid, besylic acid, fumaric acid, gluconic acid, glucuronic acid, formic acid, glutamic acid, methanesulfonic acid, ethanesulfonic acid, benzenesulfonic acid, lactic acid, oxalic acid, para-bromophenylsulfonic acid, carbonic acid, succinic acid, citric acid, benzoic acid and acetic acid, as well as related inorganic and organic acids. Such pharmaceutically acceptable salts thus include sulfate, pyrosulfate, bisulfate, sulfite, bisulfite, phosphate, monohydrogenphosphate, dihydrogenphosphate, metaphosphate, pyrophosphate, chloride, bromide, iodide, acetate, propionate, decanoate, caprylate, acrylate, formate, isobutyrate, caprate, heptanoate, propiolate, oxalate, malonate, succinate, suberate, sebacate, fumarate, maleate, butyne-1,4-dioate, hexyne-1,6-dioate, benzoate, chlorobenzoate, methylbenzoate, dinitrobenzoate, hydroxybenzoate, methoxybenzoate, phthalate, terephthalate, sulfonate, xylene sulfonate, phenylacetate, phenylpropionate, phenylbutyrate, citrate, lactate, β-hydroxybutyrate, glycolate, maleate, tartrate, methanesu1fonate, propanesulfonate, naphthalene-1-sulfonate, naphthalene-2- sulfonate, mandelate and other salts. In one embodiment, pharmaceutically acceptable acid addition salts include those formed with mineral acids such as hydrochloric acid and hydrobromic acid, and especially those formed with organic acids such as maleic acid. In some embodiments, bases commonly employed to form pharmaceutically acceptable salts of the compounds of any one of the formulae disclosed herein include hydroxides of alkali metals, including sodium, potassium, and lithium; hydroxides of alkaline earth metals such as calcium and magnesium; hydroxides of other metals, such as aluminum and zinc; ammonia, organic amines such as unsubstituted or hydroxyl-substituted mono-, di-, or tri-alkylamines, dicyclohexylamine; tributyl amine; pyridine; N-methyl, N-ethylamine; diethylamine; triethylamine; mono-, bis-, or tris-(2-OH-(C1-C6)-alkylamine), such as N,N-dimethyl-N-(2-hydroxyethyl)amine or tri-(2-hydroxyethyl)amine; N-methyl-D-glucamine; morpholine; thiomorpholine; piperidine; pyrrolidine; and amino acids such as arginine, lysine, and the like. In some embodiments, the compounds of any one of the formulae disclosed herein, or salts thereof, are substantially isolated.

The compounds of the present disclosure can be prepared using routine synthetical pathways known in the art or by following the below reaction schemes.

### EXAMPLE and COMPARATIVE EXAMPLE

All starting materials/reagents/solvents were obtained from Sigma Aldrich, Thermo Fisher, VWR, TCI Chemicals, or Oakwood chemicals and used without purification. Genipin was supplied by Herb-Sun Biotechnology.

Comparative Example 1, i.e. methyl (4aS,7aS)-7-formyl-1-hydroxy-1,4a,5,7a-tetrahydrocyclo-penta[c]-pyran-4-carboxylate, was prepared as follows:

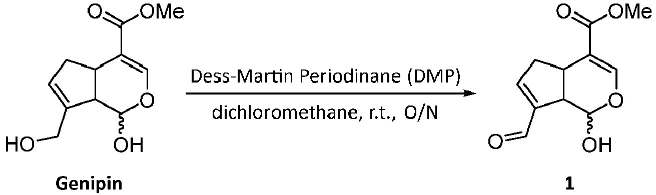

To a stirred solution of genipin (50 mmol, 1 equivalent) in dichloromethane, was added Dess-Martin periodinane (DMP) (55 mmol, 1.2 equivalent) and the mixture was stirred overnight. A saturated solution of NaHCOs and Na₂S₂O₃ was added sequentially to the reaction mixture. The resulting mixture was added to a separatory funnel and extracted with dichloromethane/water (3 times) followed by brine. The combined organic layer was dried with MgSO₄ and concentrated under reduced pressure. The resultant crude material had 10-15% hydration product. The crude mixture was then dissolved in methanol and to this was added a spatula tip of Amberlite^{®} IRA-400 resin (hydroxide form). This combination was stirred at room temperature for 15 minutes. The resin was filtered, the crude material was dry loaded onto silica and purified by flash chromatography (hexane: ethyl acetate 60:40). NMR data was consistent with previous reports (see Tetrahedron, 1993, 49, 10555-10576; Tetrahedron Letters, 1993, 34, 2621-2624).
**¹H NMR** (**400 MHz, CDCl₃**) δ 9.80 (s, 1H), 9.69 (s, 1H), 7.59-7.54 (m, 1H), 7.50 (s, 1H), 7.23 (m, 1H), 7.16 (m, 1H), 6.71 (d, J = 10.9 Hz, 1H), 5.24 (dd, J = 10.9, 2.1 Hz, 1H), 4.79 (dd, J = 8.4, 5.9 Hz, 1H), 4.57 (d, J = 6.2 Hz, 1H), 3.74 (s, 3H), 3.72 (s, 3H), 3.50 (m, 2H), 3.36-3.18 (m, 2H), 3.02-2.87 (m, 2H), 2.72 (dtd, J= 19.8, 2.8, 1.7 Hz, 1H), 2.49-2.33 (m, 1H).
**¹³C NMR** (**100 MHz, CDCl₃**) δ 191.8, 191.0, 167.6, 167.4, 161.8, 156.0, 154.7, 153.3, 144.9, 144.6, 109.9, 109.6, 95.6, 95.1, 51.5, 51.4, 48.3, 45.8, 40.8, 39.6, 37.3, 36.0;
**HRMS** (**DART**+): Calculated for C₁₁H₁₃O₅ [M+H]⁺: 225.07575 m/z, found: 225.07637 m/z

Example 1 was prepared from Comparative Example 1 using the following overall reaction scheme:

### Overall Reaction Scheme:

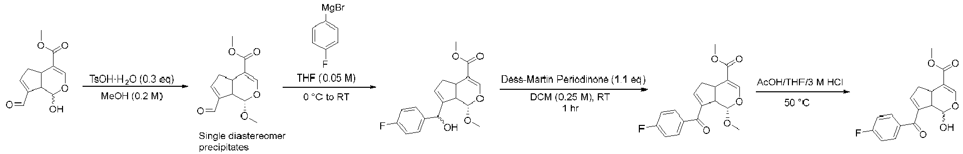

### Step 1:

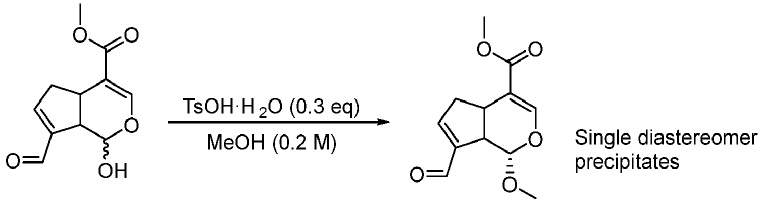

Genipin aldehyde (1 equivalent) was added to a round bottom flask and dissolved in dry methanol (0.2M). To this, was added p-toluene sulfonic acid monohydrate (0.3 eq) and the reaction was allowed to stir for 68 hrs, or until complete conversion was observed by TLC. The methanol was removed in vacuo, and the residue was redissolved in dichloromethane. The solution was run through a silica plug and flushed with dichloromethane to remove the acid catalyst. The dichloromethane is removed via rotary evaporation, and then diethyl ether/hexane was added to the residue resulting in a white precipitate. The precipitate is filtered via Buchner funnel and washed with cold ether/hexane.

### Step 2:

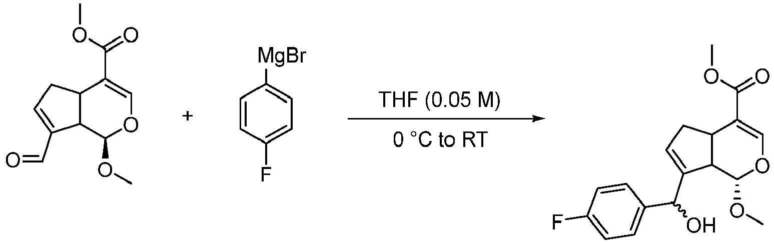

The crude aldehyde from Step 1 was transferred to a 1 L Schlenk flask. To this, was added THF (0.05 M) and a magnetic stir bar. The flask was cooled to 0 °C and then the Grignard reagent (0.99 equivalents) was added dropwise at 0 °C and warmed to room temperature. The reaction was monitored by TLC and was complete after 90 minutes. Once complete, the reaction was cooled to 0 °C and quenched with 1 M HCl. The quenched solution was allowed to stir for 30 minutes. The THF/1 M HCl solution was poured into a separatory funnel with ethyl acetate and water. The aqueous phase was washed 3x with ethyl acetate. The combined organic phase was dried over sodium sulfate and concentrated via rotary evaporation. The crude reaction mixture was dry loaded onto silica and purified via flash column chromatography (EtOAc/Hexane: 0:100 → 25:75). The resulting purified material may still be a mixture of diastereomers. The two diastereomers can be separated by a 2^{nd} column with diethyl ether/hexane (20:80 → 40:60).

### Step 3:

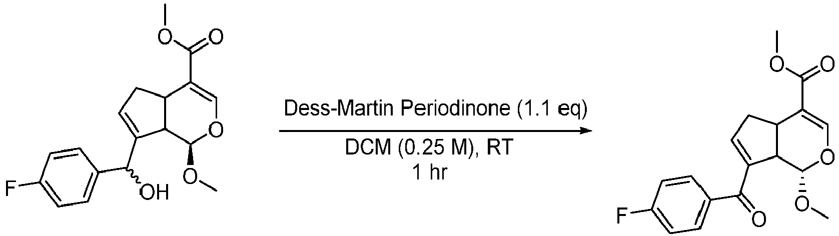

To a stirring solution of the alcohol (1 equivalent) in dichloromethane (0.25 M) was added DMP (1.1 equivalent) at room temperature. The reaction was monitored by TLC over 60 minutes. Once complete, solvent was evaporated under reduced pressure and the residue was washed with saturated sodium bicarbonate and sodium thiosulfate (Na₂S₂O₃) in a separatory funnel with diethyl ether. The organic phase was dried over sodium sulfate, concentrated, and isolated via flash column chromatography.

### Step 4:

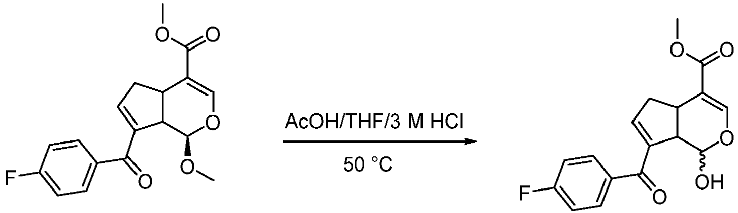

The isolated material from Step 3 was dissolved in a mixture of acetic acid, THF and 3 M HCl at an overall concentration of 0.07 M. The ratio of each was 30% AcOH, 50 % THF, and 20 % 3M HCl. The mixture was heated to 50 °C for 5 days. Once complete, the mixture was quenched with saturated sodium bicarbonate solution and checked with pH paper until neutral/basic. The mixture was then partitioned between ethyl acetate and water in a separatory funnel. The organic phase was collected and dried with sodium sulfate and concentrated in vacuo. The crude material was isolated by flash column chromatography.

### Major Diastereomer Characterization

**¹H NMR** (**400 MHz, CDC13**) δ 7.88-7.82 (m, 2H, Aryl CH), 7.64 (1H), 7.23-7.16 (m, 2H, Aryl CH), 6.89 (broad t, J = 2.7 Hz, 1H), 6.43 (d, J = 4.0 Hz, 1H, O*H*), 4.92 (broad dd, J = 8.5, 4.0 Hz, 1H), 3.78 (s, 3H), 3.39 (m, 1H), 3.29 (ddd, J = 19.0, 8.3, 3.2 Hz, 1H), 3.04 (m, 1H), 2.44 (ddt, J = 19.0, 9.5, 2.1 Hz, 1H).
**¹³C NMR** (**101 MHz, CDC13**) δ 194.7, 167.7, 167.3, 164.7, 153.4, 152.0, 141.6, 132.3, 132.2, 116.1, 115.8, 109.7, 95.9, 51.5, 48.3, 41.4, 36.3.

Genipin and the compounds of Example 1 and Comparative Example 1 were converted to their corresponding lysine conjugates according to the following procedure:
A 0.05 M solution of reactive dye precursor in MeOH was made and allowed to briefly stir in a small vial equipped with a stir bar. Two eq. of L-lysine was added, and the reaction mixture was loosely capped and allowed to stir at room temperature for 24 hours. Once complete, the solvent was removed in vacuo and further dried using high vacuum.

Example 1 provided an intense and brilliant yellow whereas the Comparative Example 1 was yellow but pale in comparison.

### LIST OF EMBODIMENTS

The following further embodiments also pertain to the present disclosure:
1. A topical composition comprising a compound of formula (I), 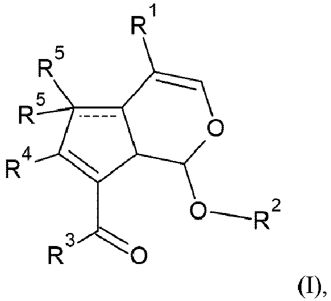 or a tautomer and/or a pharmaceutically acceptable salt thereof,
   wherein:
   R¹ represents an optionally substituted C₁-C₃₀ moiety;
   R² represents hydrogen or a protective group hydrolysable under physiological conditions after application of the topical composition onto skin;
   R³ represents an optionally substituted C₁-C₃₀ moiety;
   R⁴ represents hydrogen or an optionally substituted C₁-C₃₀ moiety;
   wherein 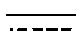 represents a single bond and both R⁵ independently from each other represent hydrogen or an optionally substituted C₁-C₃₀ moiety or wherein 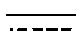 represents a double bond and one of R⁵ is absent and the other of R⁵ represents hydrogen or an
   optionally substituted C₁-C₃₀ moiety; and
   an excipient suitable for topical administration.
2. The topical composition according to embodiment 1, wherein the optionally substituted C₁-C₃₀ moiety represented by R¹ is a C₁-C₃₀ moiety comprising 1 to 30, more specifically 1 to 16, and in particular 1 to 12, carbon atoms; 0 to 12, more specifically 0 to 8, and in particular 0 to 6 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms.
3. The topical composition according to embodiment 1 or embodiment 2, wherein R¹ represents -COOH, R⁶ or -C(=O)-R⁶, wherein R⁶ represents optionally substituted C₁-C₁₂-alkyl, C₂-C₁₂-alkenyl, C₂-C₁₂-alkinyl, C₆-C₁₆-aryl or C₃-C₁₂-heteroaryl, O-C₁-C₁₂-alkyl, O-C₂-C₁₂-alkenyl, O-C₂-C₁₂-alkinyl, O-C₆-C₁₆-aryl or O-C₃-C₁₂-heteroaryl.
4. The topical composition according to any preceding embodiment, wherein R¹ represents COOH, C(=O)-O-C₁-C₁₂-alkyl or C(=O)-O-C₆-C₁₂-aryl, and in particular COOH or C(=O)-O-C₁-C₄-alkyl.
5. The topical composition according to any preceding embodiment, wherein the compound of formula (I) is a compound of formula (II), 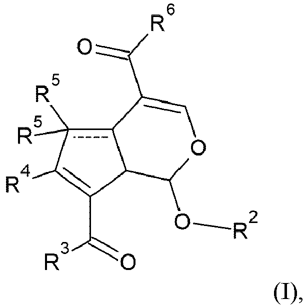 or a tautomer and/or a pharmaceutically acceptable salt thereof,
   wherein R⁶ represents hydroxyl or an optionally substituted C₁-C₃₀ moiety; and wherein R², R³, R⁴, R⁵ and 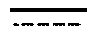 are defined as indicated in embodiment 1.
6. The topical composition according to any preceding embodiment, wherein R² represents hydrogen or a C₁-C₆ acyl, and in particular hydrogen.
7. The topical composition according to any preceding embodiment, wherein R³ represents an optionally substituted C₁-C₁₂-alkyl, optionally substituted C₂-C₁₂-alkenyl, optionally substituted C₂-C₁₂-alkinyl, optionally substituted C₆-C₁₆-aryl or optionally substituted C₃-C₁₂-heteroaryl.
8. The topical composition according to any preceding embodiment, wherein R³ represents:
   a) C₁-C₁₂-alkyl which is optionally substituted with the proviso that the optional substituents contain, in total, between 0 and 12, more specifically 0 to 8, and in particular 0 to 6, carbon atoms; between 0 to 8, more specifically 0 to 6, and in particular 0 to 4 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms;
   b) C₁-C₁₂-alkenyl which is optionally substituted with the proviso that the optional substituents contain, in total, between 0 and 12, more specifically 0 to 8, and in particular 0 to 6, carbon atoms; between 0 to 8, more specifically 0 to 6, and in particular 0 to 4 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms;
   c) C₁-C₁₂-alkinyl which is optionally substituted with the proviso that the optional substituents contain, in total, between 0 and 12, more specifically 0 to 8, and in particular 0 to 6, carbon atoms; between 0 to 8, more specifically 0 to 6, and in particular 0 to 4 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms;
   d) C₆-C₁₆-aryl which is optionally substituted with the proviso that the optional substituents contain, in total, between 0 and 12, more specifically 0 to 8, and in particular 0 to 6, carbon atoms; between 0 to 8, more specifically 0 to 6, and in particular 0 to 4 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms; or
   e) C₃-C₁₂-heteroaryl, comprising one, two, three or four heteroatoms selected from the group consisting of O, N and S, wherein the C₃-C₁₂-heteroaryl is optionally substituted with the proviso that the optional substituents contain, in total, between 0 and 12, more specifically 0 to 8, and in particular 0 to 6, carbon atoms; between 0 to 8, more specifically 0 to 6, and in particular 0 to 4 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms.
9. The topical composition according to any preceding embodiment, wherein R³ represents C₆-C₁₆-aryl which is optionally substituted by 1 to 5, more specifically 1, 2 or 3 substituent(s), and in particular 1 substituent, wherein each of said substituents is independently from each other selected from the group consisting of:
   C₁-C₆-alkyl, more specifically C₁-C₄-alkyl, and in particular methyl, ethyl and tert-butyl;
   C₁-C₄-haloalkyl, more specifically C₁-C₄-fluoroalkyl, and in particular trifluoromethyl;
   O-C₁-C₆-alkyl, more specifically O-C₁-C₄-alkyl, and in particular methoxy or ethoxy;
   O-C₂-C₁₂-alkenyl, more specifically O-C₂-C₇-alkenyl, and in particular O-C₂-C₅-alkenyl;
   (C=O)-O-C₁-C₆-alkyl, more specifically (C=O)-O-C₁-C₄-alkyl;
   O-(C=O)-C₁-C₆-alkyl, more specifically O-(C=O)-C₁-C₄-alkyl;
   halogen, more specifically chloro or fluoro, and in particular fluoro; nitro;
   NR⁶₂, wherein each of R⁶ independently from each other represents hydrogen, C₁-C₆ alkyl or C₆-C₁₂ aryl;
   NH(CO)R⁶, wherein R⁶ represents C₁-C₆ alkyl or C₆-C₁₂ aryl;
   (C=O)NR⁶₂, wherein each of R⁶ independently from each other represents hydrogen, C₁-C₆ alkyl or C₆-C₁₂ aryl; and
   S-C₁-C₆-alkyl, more specifically S-C₁-C₄-alkyl, and in particular methylthio or ethylthio.
10. The topical composition according to any preceding embodiment, wherein R³ represents C₆-C₁₆-aryl which is optionally substituted by 1 to 5, more specifically 1, 2 or 3 substituent(s), and in particular 1 substituent, wherein each of said substituents is independently from each other selected from the group consisting of:
   C₁-C₆-alkyl, more specifically C₁-C₄-alkyl, and in particular methyl, ethyl and tert-butyl;
   C₁-C₄-haloalkyl, more specifically C₁-C₄-fluoroalkyl, and in particular trifluoromethyl;
   O-C₁-C₆-alkyl, more specifically O-C₁-C₄-alkyl, and in particular methoxy or ethoxy;
   halogen, more specifically chloro or fluoro, and in particular fluoro; and nitro.
11. The topical composition according to any preceding embodiment, wherein R³ represents phenyl which is substituted by 1, 2 or 3 substituent(s), wherein each of said substituents is independently from each other selected from the group consisting of:
   C₁-C₆-alkyl, more specifically C₁-C₄-alkyl, and in particular methyl, ethyl and tert-butyl;
   C₁-C₄-haloalkyl, more specifically C₁-C₄-fluoroalkyl, and in particular trifluoromethyl;
   O-C₁-C₆-alkyl, more specifically O-C₁-C₄-alkyl, and in particular methoxy or ethoxy;
   halogen, more specifically chloro or fluoro, and in particular fluoro; and nitro.
12. The topical composition according to any preceding embodiment, wherein R³ represents phenyl which is substituted by 1, 2 or 3 fluorine atoms and/or 1, 2 or 3 CF₃ groups, more specifically wherein R³ represents phenyl which is substituted by 1 fluorine atom or 1 CF₃ group, in particular by 1 fluorine atom in the para-position, or by 2 fluorine atoms or 2 CF₃ groups, in particular by two fluorine atoms or by two CF₃ groups in the meta-position.
13. The topical composition according to any preceding embodiment, wherein R³ represents one of the following moieties: 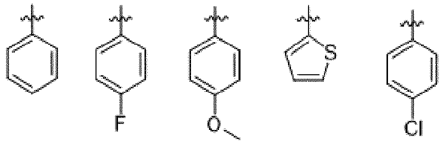 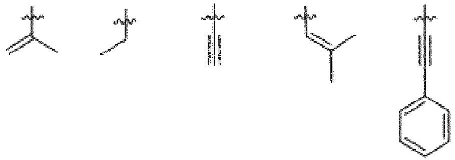 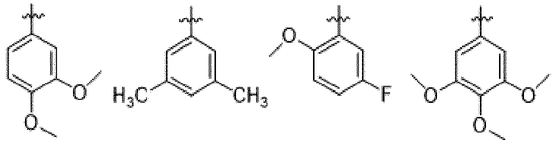 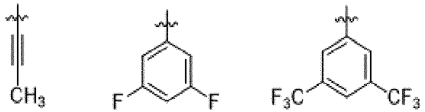
14. The topical composition according to any preceding embodiment, wherein the optionally substituted C₁-C₃₀ moiety represented by R⁴ is a C₁-C₃₀ moiety comprising 1 to 30, more specifically 1 to 16, and in particular 1 to 12, carbon atoms; 0 to 12, more specifically 0 to 8, and in particular 0 to 6 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms.
15. The topical composition according to any preceding embodiment, wherein R⁴ represents hydrogen, C₁-C₆ alkyl or C₆-C₁₆ aryl; more specifically hydrogen or C₁-C₄ alkyl; and in particular hydrogen.
16. The topical composition according to any preceding embodiment, wherein 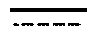 represents a single bond and both R⁵ independently from each other represent hydrogen, C₁-C₆ alkyl or C₆-C₁₂ aryl; more specifically hydrogen or C₁-C₄ alkyl; and in particular hydrogen.
17. The topical composition according to any preceding embodiment, wherein 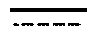 represents a double bond and one R⁵ is absent and the other R⁵ represents hydrogen, C₁-C₆ alkyl or C₆-C₁₆ aryl; more specifically hydrogen or C₁-C₄ alkyl; and in particular hydrogen.
18. The topical composition according to any preceding embodiment, wherein the compound of formula (I) is a compound of formula (III), 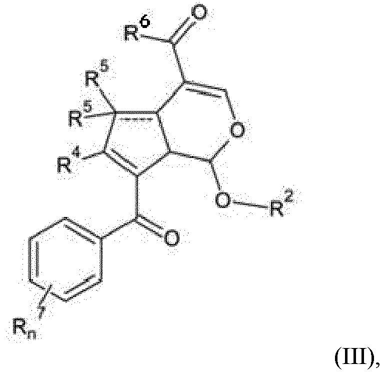
   wherein R², R⁴, R⁵ and R⁶ are as defined in any one of embodiments 1 to 17, wherein 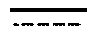 is as defined in embodiment 1, and wherein Rₙ⁷ represents n substituents R⁷,
   wherein n is an integer between 0 and 5, more specifically between 0 and 3, and in particular 1, and wherein n being 0 means that R⁷ is absent,
   wherein independently from each other R⁷ represents a moiety selected from the group consisting of:
      C₁-C₆-alkyl, more specifically C₁-C₄-alkyl, and in particular methyl, ethyl and tert-butyl;
      C₁-C₄-haloalkyl, more specifically C₁-C₄-fluoroalkyl, and in particular trifluoromethyl;
      O-C₁-C₆-alkyl, more specifically O-C₁-C₄-alkyl, and in particular methoxy or ethoxy;
      O-C₂-C₁₂-alkenyl, more specifically O-C₂-C₇-alkenyl, and in particular O-C₂-C₅-alkenyl;
      (C=O)-O-C₁-C₆-alkyl, more specifically (C=O)-O-C₁-C₄-alkyl;
      O-(C=O)-C₁-C₆-alkyl, more specifically O-(C=O)-C₁-C₄-alkyl;
      halogen, more specifically chloro or fluoro, and in particular fluoro; nitro;
      NR⁶₂, wherein each of R⁶ independently from each other represents hydrogen, C₁-C₆ alkyl or C₆-C₁₂ aryl;
      NH(CO)R⁶, wherein R⁶ represents C₁-C₆ alkyl or C₆-C₁₂ aryl;
      (C=O)NR⁶₂, wherein each of R⁶ independently from each other represents hydrogen, C₁-C₆ alkyl or C₆-C₁₂ aryl; and
      S-C₁-C₆-alkyl, more specifically S-C₁-C₄-alkyl, and in particular methylthio or ethylthio.
19. The topical composition according to embodiment 18, wherein n is 1, 2 or 3 and wherein the R⁷ substituent(s) is/are in the ortho- and/or para-position(s); in particular wherein n is 1 and R⁷ is in ortho- or para-position.
20. The topical composition according to any one of embodiments 18 to 19, wherein, independently from each other, R⁷ represents a moiety selected from the group consisting of: O-C₁-C₆-alkyl, more specifically O-C₁-C₄-alkyl, and in particular methoxy or ethoxy; halogen, more specifically chloro or fluoro, and in particular fluoro; C₁-C₄-haloalkyl, more specifically C₁-C₄-fluoroalkyl, and in particular trifluoromethyl; and nitro.
21. The topical composition according to any preceding embodiment, wherein the compound of formula (I) is a compound of formula (IV), 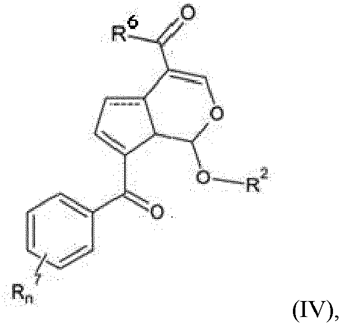 wherein R² and R⁶ are as defined in any one of embodiments 1 to 17, wherein 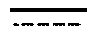 is as defined in embodiment 1, and wherein Rₙ⁷ is as defined in any one of embodiments 18 to 20.
22. The topical composition according to any preceding embodiment, wherein the compound of formula (I) is a compound of formula (V), 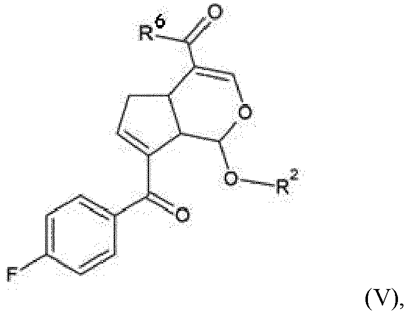 wherein R² and R⁶ are as defined in any one of embodiments 1 to 17.
23. The topical composition according to any preceding embodiment, wherein the composition is in the form of a solid, a liquid, an ink, a gel, a patch for transferring the composition onto the skin, or a substrate carrier comprising the topical composition.
24. The topical composition according to any preceding embodiment, wherein the excipient suitable for topical administration comprises one or more excipients selected from water, ethanol, isopropanol, n-propanol, ethylene glycol, diethylene glycol, a propylene glycol, glycerol, and mixtures thereof.
25. The topical composition according to any preceding embodiment, wherein the composition comprises a thickening agent and/or a film-forming agent.
26. The topical composition according to any preceding embodiment, wherein the compound of formula (I), after conversion with lysine to a corresponding compound of formula (VI), 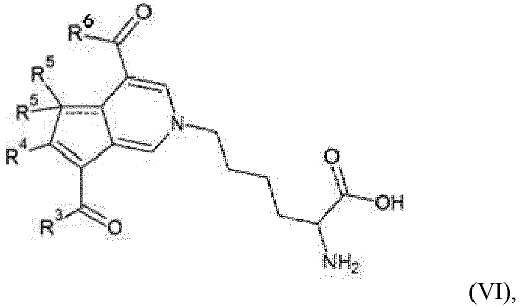
   wherein R³, R⁴, R⁵ and R⁶ are as defined in any one of embodiments 1 to 17, and wherein 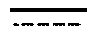 is as defined in embodiment 1,
   absorbs light at a wavelength between about 380 nm and about 565 nm, more specifically between about 380 nm and about 568 nm, and in particular between about 380 nm and about 570 nm;
   wherein the corresponding compound of formula (VI) optionally also absorbs light at a wavelength between about 590 nm and about 750 nm, more specifically between about 587 nm and about 750 nm, and in particular between about 585 nm and about 750 nm; and wherein the corresponding compound of formula (VI) optionally does not absorb light at a wavelength between about 566 nm and about 589 nm, more specifically between about 569 nm and about 586 nm, and in particular between about 571 nm and about 584 nm.
27. The topical composition according to any preceding embodiment, wherein the composition further comprises genipin or a genipin derivative, more specifically in relative molar amounts of 0.05 to 20 with respect to the amount of the compound of formula (I).
28. The topical composition according to any preceding embodiment, wherein the composition further comprises an antioxidant.
29. A storage unit comprising the topical composition according to any preceding embodiment, wherein the storage unit is protected against ingress of oxygen or contains an oxygen scavenger.
30. Process of preparing the topical composition of any one of embodiments 1 to 28, the process comprising mixing a compound of formula (I), as defined in any of embodiments 1 to 28, with an excipient suitable for topical administration.
31. Process of mixing a compound of formula (I), as defined in any of embodiments 1 to 28, with genipin or a genipin derivative.

## Claims

1. A topical composition comprising a compound of formula (I), 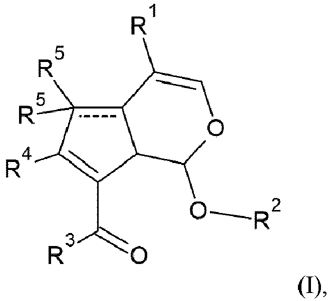 or a tautomer and/or a pharmaceutically acceptable salt thereof,
wherein:
R¹ represents an optionally substituted C₁-C₃₀ moiety;
R² represents hydrogen or a protective group hydrolysable under physiological conditions after application of the topical composition onto skin;
R³ represents an optionally substituted C₁-C₃₀ moiety;
R⁴ represents hydrogen or an optionally substituted C₁-C₃₀ moiety;
wherein 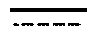 represents a single bond and both R⁵ independently from each other represent hydrogen or an optionally substituted C₁-C₃₀ moiety or wherein 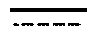 represents a double bond and one of R⁵ is absent and the other of R⁵ represents hydrogen or an
optionally substituted C₁-C₃₀ moiety; and
an excipient suitable for topical administration.

2. The topical composition according to claim 1, wherein the optionally substituted C₁-C₃₀ moiety represented by R¹ is a C₁-C₃₀ moiety comprising 1 to 30, more specifically 1 to 16, and in particular 1 to 12, carbon atoms; 0 to 12, more specifically 0 to 8, and in particular 0 to 6 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms.

3. The topical composition according to claim 1 or claim 2, wherein R¹ represents -COOH, R⁶ or -C(=O)-R⁶, wherein R⁶ represents optionally substituted C₁-C₁₂-alkyl, C₂-C₁₂-alkenyl, C₂-C₁₂-alkinyl, C₆-C₁₆-aryl or C₃-C₁₂-heteroaryl, O-C₁-C₁₂-alkyl, O-C₂-C₁₂-alkenyl, O-C₂-C₁₂-alkinyl, O-C₆-C₁₆-aryl or O-C₃-C₁₂-heteroaryl.

4. The topical composition according to any preceding claim, wherein the compound of formula (I) is a compound of formula (II), 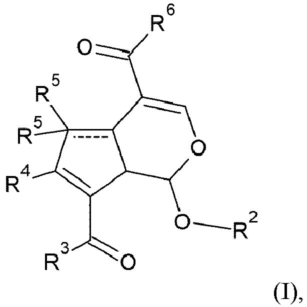 or a tautomer and/or a pharmaceutically acceptable salt thereof,
wherein R⁶ represents hydroxyl or an optionally substituted C₁-C₃₀ moiety; and wherein R², R³, R⁴, R⁵ and 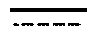 are defined as indicated in claim 1.

5. The topical composition according to any preceding embodiment, wherein R² represents hydrogen or a C₁-C₆ acyl, and in particular hydrogen.

6. The topical composition according to any preceding claim, wherein R³ represents an optionally substituted C₁-C₁₂-alkyl, optionally substituted C₂-C₁₂-alkenyl, optionally substituted C₂-C₁₂-alkinyl, optionally substituted C₆-C₁₆-aryl or optionally substituted C₃-C₁₂-heteroaryl.

7. The topical composition according to any preceding claim, wherein R³ represents:
a) C₁-C₁₂-alkyl which is optionally substituted with the proviso that the optional substituents contain, in total, between 0 and 12, more specifically 0 to 8, and in particular 0 to 6, carbon atoms; between 0 to 8, more specifically 0 to 6, and in particular 0 to 4 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms;
b) C₁-C₁₂-alkenyl which is optionally substituted with the proviso that the optional substituents contain, in total, between 0 and 12, more specifically 0 to 8, and in particular 0 to 6, carbon atoms; between 0 to 8, more specifically 0 to 6, and in particular 0 to 4 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms;
c) C₁-C₁₂-alkinyl which is optionally substituted with the proviso that the optional substituents contain, in total, between 0 and 12, more specifically 0 to 8, and in particular 0 to 6, carbon atoms; between 0 to 8, more specifically 0 to 6, and in particular 0 to 4 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms;
d) C₆-C₁₆-aryl which is optionally substituted with the proviso that the optional substituents contain, in total, between 0 and 12, more specifically 0 to 8, and in particular 0 to 6, carbon atoms; between 0 to 8, more specifically 0 to 6, and in particular 0 to 4 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms; or
e) C₃-C₁₂-heteroaryl, comprising one, two, three or four heteroatoms selected from the group consisting of O, N and S, wherein the C₃-C₁₂-heteroaryl is optionally substituted with the proviso that the optional substituents contain, in total, between 0 and 12, more specifically 0 to 8, and in particular 0 to 6, carbon atoms; between 0 to 8, more specifically 0 to 6, and in particular 0 to 4 oxygen atoms; 0 to 8, more specifically 0 to 6, and in particular 0 to 4 nitrogen atoms; 0 to 6, more specifically 0 to 4, and in particular 0 to 3 sulfur atoms; and 0 to 10, more specifically 0 to 8, and in particular 0 to 6 halogen atoms.

8. The topical composition according to any preceding claim, wherein R³ represents C₆-C₁₆-aryl which is optionally substituted by 1 to 5, more specifically 1, 2 or 3 substituent(s), and in particular 1 substituent, wherein each of said substituents is independently from each other selected from the group consisting of:
C₁-C₆-alkyl, more specifically C₁-C₄-alkyl, and in particular methyl, ethyl and tert-butyl;
C₁-C₄-haloalkyl, more specifically C₁-C₄-fluoroalkyl, and in particular trifluoromethyl;
O-C₁-C₆-alkyl, more specifically O-C₁-C₄-alkyl, and in particular methoxy or ethoxy;
O-C₂-C₁₂-alkenyl, more specifically O-C₂-C₇-alkenyl, and in particular O-C₂-C₅-alkenyl;
(C=O)-O-C₁-C₆-alkyl, more specifically (C=O)-O-C₁-C₄-alkyl;
O-(C=O)-C₁-C₆-alkyl, more specifically O-(C=O)-C₁-C₄-alkyl;
halogen, more specifically chloro or fluoro, and in particular fluoro; nitro;
NR⁶₂, wherein each of R⁶ independently from each other represents hydrogen, C₁-C₆ alkyl or C₆-C₁₂ aryl;
NH(CO)R⁶, wherein R⁶ represents C₁-C₆ alkyl or C₆-C₁₂ aryl;
(C=O)NR⁶₂, wherein each of R⁶ independently from each other represents hydrogen, C₁-C₆ alkyl or C₆-C₁₂ aryl; and
S-C₁-C₆-alkyl, more specifically S-C₁-C₄-alkyl, and in particular methylthio or ethylthio.

9. The topical composition according to any preceding claim, wherein R³ represents C₆-C₁₆-aryl which is optionally substituted by 1 to 5, more specifically 1, 2 or 3 substituent(s), and in particular 1 substituent, wherein each of said substituents is independently from each other selected from the group consisting of:
C₁-C₆-alkyl, more specifically C₁-C₄-alkyl, and in particular methyl, ethyl and tert-butyl;
C₁-C₄-haloalkyl, more specifically C₁-C₄-fluoroalkyl, and in particular trifluoromethyl;
O-C₁-C₆-alkyl, more specifically O-C₁-C₄-alkyl, and in particular methoxy or ethoxy;
halogen, more specifically chloro or fluoro, and in particular fluoro; and nitro.

10. The topical composition according to any preceding claim, wherein R³ represents phenyl which is substituted by 1, 2 or 3 substituent(s), wherein each of said substituents is independently from each other selected from the group consisting of:
C₁-C₆-alkyl, more specifically C₁-C₄-alkyl, and in particular methyl, ethyl and tert-butyl;
C₁-C₄-haloalkyl, more specifically C₁-C₄-fluoroalkyl, and in particular trifluoromethyl;
O-C₁-C₆-alkyl, more specifically O-C₁-C₄-alkyl, and in particular methoxy or ethoxy;
halogen, more specifically chloro or fluoro, and in particular fluoro; and nitro.

11. The topical composition according to any preceding claim, wherein the compound of formula (I) is a compound of formula (III), 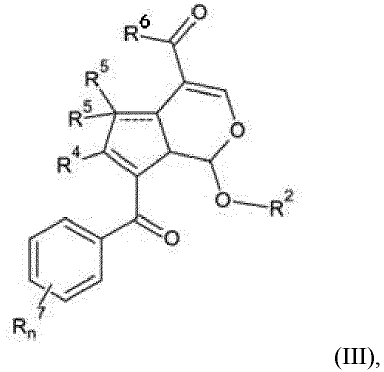
wherein R², R⁴, R⁵ and R⁶ are as defined in any one of claims 1 to 17, wherein 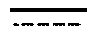 is as defined in claim 1, and wherein Rₙ⁷ represents n substituents R⁷,
wherein n is an integer between 0 and 5, more specifically between 0 and 3, and in particular 1, and wherein n being 0 means that R⁷ is absent,
wherein independently from each other R⁷ represents a moiety selected from the group consisting of:
C₁-C₆-alkyl, more specifically C₁-C₄-alkyl, and in particular methyl, ethyl and tert-butyl;
C₁-C₄-haloalkyl, more specifically C₁-C₄-fluoroalkyl, and in particular trifluoromethyl;
O-C₁-C₆-alkyl, more specifically O-C₁-C₄-alkyl, and in particular methoxy or ethoxy;
O-C₂-C₁₂-alkenyl, more specifically O-C₂-C₇-alkenyl, and in particular O-C₂-C₅-alkenyl;
(C=O)-O-C₁-C₆-alkyl, more specifically (C=O)-O-C₁-C₄-alkyl;
O-(C=O)-C₁-C₆-alkyl, more specifically O-(C=O)-C₁-C₄-alkyl;
halogen, more specifically chloro or fluoro, and in particular fluoro; nitro;
NR⁶₂, wherein each of R⁶ independently from each other represents hydrogen, C₁-C₆ alkyl or C₆-C₁₂ aryl;
NH(CO)R⁶, wherein R⁶ represents C₁-C₆ alkyl or C₆-C₁₂ aryl;
(C=O)NR⁶₂, wherein each of R⁶ independently from each other represents hydrogen, C₁-C₆ alkyl or C₆-C₁₂ aryl; and
S-C₁-C₆-alkyl, more specifically S-C₁-C₄-alkyl, and in particular methylthio or ethylthio.

12. The topical composition according to any preceding claim, wherein the compound of formula (I) is a compound of formula (IV), 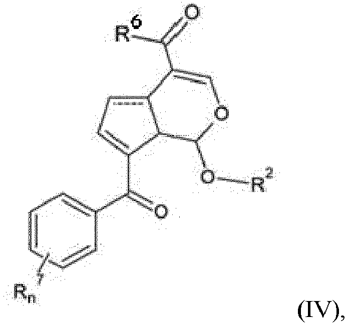 wherein R² and R⁶ are as defined in any one of claims 1 to 10, wherein 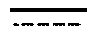 is as defined in claim 1, and wherein Rₙ⁷ is as defined in claim 11.

13. The topical composition according to any preceding claim, wherein the compound of formula (I) is a compound of formula (V), 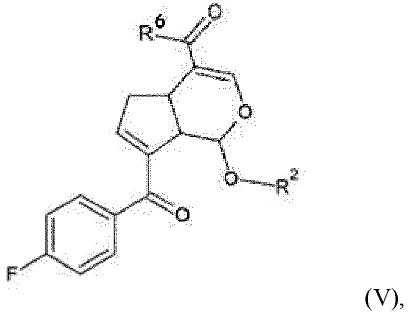 wherein R² and R⁶ are as defined in any one of claims 1 to 10.

14. The topical composition according to any preceding claim, wherein the compound of formula (I), after conversion with lysine to a corresponding compound of formula (VI), 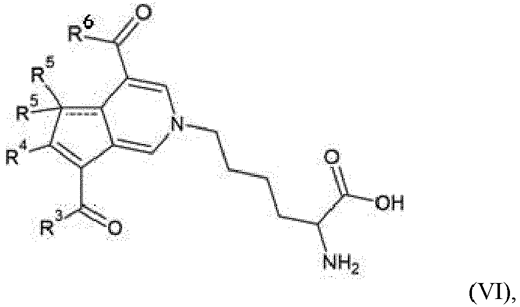
wherein R³, R⁴, R⁵ and R⁶ are as defined in any one of claims 1 to 10, and wherein 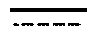 is as defined in claim 1,
absorbs light at a wavelength between about 380 nm and about 565 nm, more specifically between about 380 nm and about 568 nm, and in particular between about 380 nm and about 570 nm;
wherein the corresponding compound of formula (VI) optionally also absorbs light at a wavelength between about 590 nm and about 750 nm, more specifically between about 587 nm and about 750 nm, and in particular between about 585 nm and about 750 nm; and wherein the corresponding compound of formula (VI) optionally does not absorb light at a wavelength between about 566 nm and about 589 nm, more specifically between about 569 nm and about 586 nm, and in particular between about 571 nm and about 584 nm.

15. Process of mixing a compound of formula (I), as defined in any of claims 1 to 14, with genipin or a genipin derivative.
